# EUROPÄISCHE PATENTSCHRIFT

(11) **EP 1 343 770 B1**
(45) Veröffentlichungstag und Bekanntmachung des Hinweises auf die Patenterteilung: **06.05.2009**
(21) Anmeldenummer: 01991788.9
(22) Anmeldetag: 05.12.2001
(51) Int. Cl.: C07D 257/00

(54) **LITHIUM-KOMPLEXE VON N-(1-HYDROXYMETHYL-2,3-DIHYDROXYPROPYL)-1,4,7-TRISCARBOXYMETHYL-1,4,7,10-TETRAAZACYCLODODECAN, DEREN HERSTELLUNG UND VERWENDUNG**
LITHIUM COMPLEXES OF N-(1-HYDROXYMETHYL-2,3-DIHYDROXYPROPYL)-1,4,7-TRISCARBOXYMETHYL-1,4,7,10-TETRAAZACYCLODODECANE, PRODUCTION AND USE THEREOF
COMPLEXES DE LITHIUM DU N-(1-HYDROXYMETHYL-2,3-DIHYDROXYPROPYL)-1,4,7-TRISCARBOXYMETHYL-1,4,7,10-TETRAAZACYCLODODECANE, LEUR FABRICATION ET LEUR UTILISATION

(30) Priorität: 15.12.2000 DE 10064467
(43) Veröffentlichungstag der Anmeldung: 17.09.2003
(73) Patentinhaber: Bayer Schering Pharma Aktiengesellschaft, 13353 Berlin (DE)
(72) Erfinder: PLATZEK, Johannes, 12621 Berlin (DE); BLASZKIEWICZ, Peter, 12167 Berlin (DE); PETROV, Orlin, 14199 Berlin (DE); HOFFMANN, Holger, 94300 Orizaba, Vera Cruz (MX)
(86) Internationale Anmeldenummer: PCT/EP2001/014283
(87) Internationale Veröffentlichungsnummer: WO 2002/048119

(56) Entgegenhaltungen:
- EP-A- 0 448 191
- WO-A-98/55467
- PLATZEK J. ET AL: "Synthesis and Structure of a New Macrocyclic Polyhydroxylated Gadolinium Chelate Used as Contrast Agent for Magnetic Resonance Imaging" INORG. CHEM. , Bd. 36, Nr. 26, 1997, Seiten 6086-6093, XP002199426 in der Anmeldung erwähnt

## Beschreibung

Die Erfindung betrifft den in den Patentansprüchen gekennzeichneten Gegenstand, d.h. Lithium-Komplexe von N-(1-Hydroxymethyl-2,3-dihydroxypropyl)-1,4,7-triscarboxymethyl-1,4,7,10-tetraazacyclododecan deren Herstellung und Verwendung.

Wegen ihrer Bedeutung als bildgebende Diagnostika, insbesondere MRI-Diagnostika, ist die Herstellung von Metallkomplexen, insbesondere des Gadoliniumkomplexes, des N-(1-Hydroxymethyl-2,3-dihydroxypropyl)-1,4,7-triscarboxymethyl-1,4,7,10-tetraazacyclododecans "GADOBUTROL" (DE 4009119) auf verschiedenen Wegen versucht worden. Trotz erzielter Fortschritte gegenüber den ursprünglichen Verfahren besteht weiterhin ein Bedarf für vor allem im Betriebsmaßstab durchzuführende umweltfreundlichere und kostengünstigere Synthesemöglichkeiten.

Diese Aufgabe wird durch die vorliegende Erfindung gelöst.

Es wurde gefunden, dass überraschenderweise die erfindungsgemäßen kristallinen Komplexbildner der allgemeinen Formel 1, 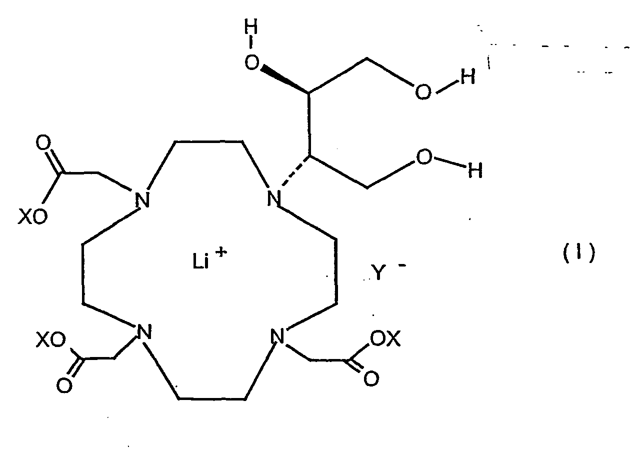 worin die drei X insgesamt für n Lithiumionen und m Wasserstoffatome stehen, wobei n und m jeweils die Zahlen 0 bis 3, bevorzugt 0,8-2,2 bedeuten und die Summe n und m = 3 ist und Y die Bedeutung von Chlorid und Bromid hat, d.h. Lithium-Komplexe von N-(1-Hydroxymethyl-2,3-dihydroxypropyl)-1,4,7-triscarboxymethyl-1,4,7,10-tetraazacyclododecan sehr gut geeignet sind den Gadoliniumkomplex GADOBUTROL auf eine Weise herzustellen, die dem nächstliegenden Stand der Technik (Inorg. Chem. 1997, 36, 6086-6093 und DE 19724186.7) deutlich überlegen ist.

Bevorzugt steht Y für Chlorid, ein X für ein Lithiumion und die restlichen beiden stehen für Wasserstoffatome.

Ausgehend vom N-(6-Hydroxy-2,2-dimethyl-1,3-dioxepan-5-yl)-1,4,7,10-tetraazacyclododecan-LiCl-bzw. LiBr-Komplex (DE 19724186.7) wird die erfindungsgemäße Verbindung unter Verwendung von Chlor- oder Bromessigsäure, Lithiumhydroxid und Chlorwasserstoffsäure oder Bromwasserstoffsäure in Wasser hergestellt und aus einem Ethanol-WasserGemisch kristallisiert. Der kristalline Komplexbildner wird in Wasser mit Gadolinium komplexiert und der Komplex durch Kristallisation aus Ethanol/Wasser ohne Verwendung von Ionenaustauscher salzfrei isoliert.

Die Vorteile dieses Verfahrens sind:
Der Komplexbildner wird kristallin isoliert und dabei in sehr reiner Form erhalten.

Das Verfahren zur Herstellung des kristallinen Komplexbildners vermeidet durch die Verwendung von Lithiumhydroxid an Stelle von Natriumhydroxid (DE 197241867) die aufwendige- Isolierung des apparatekorrosiven Natriumchlorids aus stark saurer methanolisch-wässriger Lösung. Das Lithiumchlorid bzw. Lithiumbromid, das beim erfindungsgemäßen Verfahren statt Natriumchlorid entsteht, bleibt bei der Kristallisation des Komplexbildners in der schwach sauren ethanolisch-wässrigen Mutterlauge. Das Lithium kann aus dieser Mutterlauge durch Behandlung mit einem Anionenaustauscher als

Lithiumhydroxid zurückgewonnen werden. Die Bilanz des Herstellprozesses wird hinsichtlich der Abfälle wesentlich günstiger.

Das Gadolinium kann bei der Komplexierung zur Herstellung von GADOBUTROL genau dosiert werden. Dadurch verringert sich die Menge des Produktionsabfalls, der Gadolinium enthält.

Zur Entfernung von Salzen und anderen Nebenprodukten aus dem Gadolinium-Komplex sind im Gegensatz zum Verfahren des Standes der Technik keine Ionenaustauscher erforderlich. Dadurch entfallen der Betrieb einer entsprechenden technischen Anlage und die damit verbundenen Abfallprodukte.

Es entfällt das energieintensive Eindampfen der wässrigen Eluate der Ionenaustauscher.

Die erfindungsgemäßen Verbindungen werden erhalten, indem man den LiCl- bzw. LiBr-Komplex von N-(6-Hydroxy-2,2-dimethyl-1,3-dioxepan-5-yl)-1,4,7,10-tetraazacyclododecan in polaren Lösungsmitteln, wie Wasser, primären und sekundären Alkoholen, z.B. Ethanol oder Isopropanol, DMF, Dimethoxyethan, Diethylenglykoldimethylether oder Gemischen dieser Lösungsmittel, vorzugsweise in Wasser, mit Chlor- oder Bromessigsäure, bevorzugt mit Chloressigsäure, und Lithiumhydroxid bei Temperaturen zwischen 40 und 150° C, bevorzugt bei 40 bis 90° C, einem pH-Wert zwischen 8 und 14, bevorzugt bei pH-Wert 9 bis 13, innerhalb von 0,5 bis 24, bevorzugt 1 bis 6, Stunden umsetzt.

Zur Isolierung des Produkts wird mit Chlorwasserstoffsäure, Bromwasserstoffsäure, Essigsäure, Trifluoressigsäure oder p-Toluolsulfonsäure, bevorzugt mit Chlorwasserstoffsäure, auf pH-Wert 3,5 bis 4,5, bevorzugt auf pH-Wert 3,8 bis 4,2, eingestellt und aus einem Gemisch von Wasser und Ethanol kristallisiert.

Zur Überführung des Komplexbildners der allgemeinen Formel I in den Gadolinium-Komplex der allgemeinen Formel II wird der Komplexbildner in Wasser gelöst, durch Zusatz von Chlorwasserstoffsäure der pH-Wert auf ca. 3.6 gestellt, die berechnete Menge Gadoliniumoxid hinzugefügt, bei 50 bis 100° C, bevorzugt bei 70 bis 100° C komplexiert und der Gadolinium-Komplex durch Zugabe von Ethanol kristallisiert.

Die Erfindung wird durch die nachfolgenden Beispiele erläutert:

### Beispiel 1

Die Herstellung des Lithium-Komplexes von (N-(1-Hydroxymethyl-2,3-dihydroxypropyl)-1,4,7-triscarboxymethyl-1,4,7,10-tetraazacydododecan:
38,5 g Chloressigsäure werden vorgelegt, in 40 g Wasser gelöst und die Lösung auf 0 bis 10° C gekühlt. Zu dieser Lösung werden 17,1 g Lithiumhydroxid-monohydrat hinzugesetzt. Diese Lösung wird einer Lösung von 41,25 g (114,95 mmol) des Lithiumchlorid-Komplexes von N-(6-Hydroxy-2,2-dimethyl-1,3-dioxepan-5-yl)-1,4,7,10-tetraazacyclododecan, gelöst in ca. 45 ml Wasser, zugesetzt. Das Gemisch wird auf ca. 65° erwärmt und bei dieser Temperatur innerhalb von 2 Stunden in Portionen insgesamt 14,6 g Lithiumhydroxid-monohydrat zugesetzt. Danach wird 1 Stunde bei 65° C nachgerührt. Die Lösung wird dann mit Salzsäure auf pH 4 angesäuert. Bei einer Innentemperatur von 65 bis 75° C werden der Lösung im Verlaufe von 75 Minuten 500 ml Ethanol zugesetzt. Die Kristallisation des Lithium-Komplexes setzt gegen Ende der Ethanolzugabe spontan ein. Nach beendeter Ethanolzugabe wird 2 Stunden am Rückfluß gekocht, dann auf Raumtemperatur gekühlt, das Kristallisat abfiltriert, mit 2x20 ml 80 %igem und 2x20 ml 90 %igem Ethanol gewaschen und bei 50° C getrocknet.

Ausbeute: 51,6 g = 93,67 mmol unter Berücksichtigung des Wassergehaltes = 81,5 % der Theorie.

Analytik:
H-NMR in D₂O: Multiplett zwischen 3,0 und 3,9 ppm für N-CH₂-CH₂-N (4x), N-CH₂-COOH (3x), N-CH (1x), CH-OH (1x) und CH₂-OH (2x).
IR (KBr, cm⁻¹): 3440, 3360, 3300, 1675, 1650, 1590, 1400
FAB-MS (Matrix NBA-Glycerin-DMSO: 451 (M+H), 45 (M+Li), 473 (M+Na)
FAB-MS (Matrix Magic Bullet) 451 (M+H), 457 (M+7)
Wassergehalt 9,45 %, Ethanolgehalt 0,0 %.

Elementaranalyse berechnet für das Gemisch: Li-Komplex + 9.45 % = 2,89 mol Wasser:

| % | C | H | Cl | Li | N | Wasser | EtOH |
|---|---|---|---|---|---|---|---|
| Theorie: | 39,25 | 7,1 | 6,44 | 2,52 | 10,17 | 9,45 | 0 |
| Gefunden | 38,82 | 6,86 | 6,83 | 2,64 | 10,17 | 9,45 | 0 |
| Differenz | -0,43 | -0,24 | 0,39 | 0,12 | 0 | 0 | 0 |

### Beispiel 2

Die Herstellung des Gadolinium-Komplexes von (N-(1-Hydroxymethyl-2,3-dihydroxypropyl)-1,4,7-triscarboxymethyl-1,4,7,10-tetraazacyclododecan (GADOBUTROL):
51,6 g (93,67 mmol unter Berücksichtigung von 9,45 % Wasser) kristalliner Lithium-Komplexes von N-(1-Hydroxymethyl-2,3-dihydroxypropyl)-1,4,7-triscarboxymethyl-1,4,7,10-tetraazacyclododecan (Beispiel 1) werden in 51 g vollentsalztem Wasser gelöst und die Lösung mit konzentrierter Salzsäure auf einen pH-Wert von 3,5 gestellt. Der Lösung werden 16,9 g Gd₂O₃ zugesetzt und die Suspension 1 Stunde bei 90° C gerührt. Die Suspension geht in eine Lösung über. Der pH-Wert dieser Lösung wird gegebenenfalls mit festem Lithiumhydroxid-monohydrat auf 7 gestellt. Bei einer Temperatur von ca. 78° C wird die Lösung im Verlauf von 2 Stunden allmählich mit 960 ml Ethanol versetzt. Es entsteht eine Suspension. Diese wird nach beendeter Zugabe des Ethanols 5 Stunden am Rückfluß gekocht. Die Suspension wird dann auf Raumtemperatur gekühlt, das Kristallisat abfiltriert, mit 100 ml Ethanol gewaschen und bei 50°C getrocknet.

Ausbeute: 51.02 g = 80.3 mmol unter Berücksichtigung des Wassergehaltes = 85.73% d. Th.

Trockenverlust 1.06 %, Wassergehalt 4.74 %.

Analyse für GADOBUTROL mit 1.7 mol Wasser = 4.82% und 0.53 mol LiCl:

| % | C | H | N | Cl | Li | Gd | Wasser | EtOH |
|---|---|---|---|---|---|---|---|---|
| Theorie: | 32.87 | 5.27 | 8.52 | 2.86 | 0.56 | 23.91 | 4.82 | 0 |
| Gefunden | 32.96 | 5.25 | 8.49 | 2.91 | 0.53 | 23.85 | 4.74 | 0 |
| Differenz | 0.09 | -0.02 | -0.03 | 0.05 | -0.03 | -0.06 | -0.08 | 0 |

### Umkristallisation zur vollständigen Entsalzung:

51.02 g GADOBUTROL, roh = 80.3 mmol unter Berücksichtigung des Wassergehaltes, werden in 47ml Wasser bei ca. 75°C gelöst und der Lösung im Verlaufe von einer Stunde 470 ml Ethanol allmählich zugefügt. Es entsteht eine Suspension. Diese wird nach beendeter Zugabe des Ethanols 2 Stunden am Rückfluß gekocht, dann auf 20°C gekühlt, 1 Stunde bei dieser Temperatur gerührt, das Kristallisat abgesaugt, mit 66ml Ethanol gewaschen und bei 50°C getrocknet.

Ausbeute: 47.08 g = 74.84 mmol unter Berücksichtigung des Wassergehaltes = 93.2 % d. Th.

Analytik: Wassergehalt 3.92%, Ethanolgehalt 0.16%. Elementaranalyse für: GADOBUTROL mit 1.35 mol Wasser = 3.86%, 0.02 mol EtOH = 0.15% und 0 mol LiCl:

| % | C | H | N | Cl | Li | Gd | Wasser | EtOH |
|---|---|---|---|---|---|---|---|---|
| Theorie: | 34.4 | 5.4 | 8.89 | 0 | 0 | 24.96 | 3.86 | 0.15 |
| Gefunden | 34.48 | 5.01 | 8.76 | 0 | <0.01 | 24.96 | 3.92 | 0.16 |
| Differenz | 0.08 | -0.39 | -0.13 | 0 | <0.01 | 0.09 | 0.06 | 0.01 |

## Patentansprüche

1. Verbindungen der allgemeinen Formel I 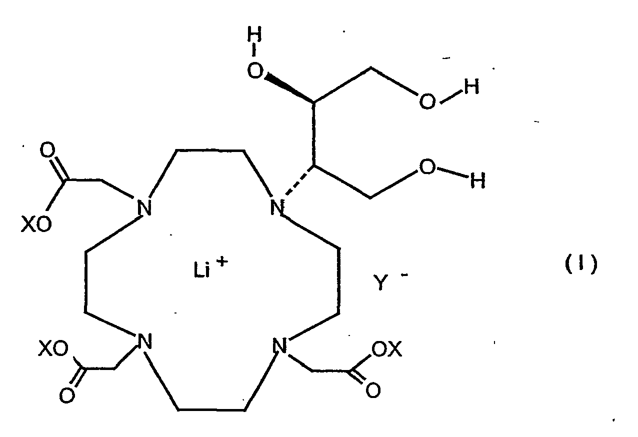 worin die drei X insgesamt für n Lithiumiorien und m Wasserstoffatome stehen, wobei n und m jeweils die Zahlen 0 bis 3, bevorzugt 0,8-2,2 bedeuten und die Summe n und m = 3 ist und Y die Bedeutung von Chlorid und Bromid hat.

2. Verfahren zur Herstellung von Verbindungen der allgemeinen Formel I gemäß Anspruch 1, **dadurch gekennzeichnet; dass** man N-(6-Hydroxy-2,2-dimethyl-1,3-dioxepan-5-yl)-1,4,7,10-tetraazacyclododecan oder dessen Komplex mit Lithiumchlorid oder Lithiumbromid mit Chlor- oder Bromessigsäure und Lithiumhydroxid in polaren Lösungsmitteln bei Temperaturen zwischen 40 und 150 ° C und einem pH-Wert zwischen 8 und 14 umsetzt.

3. Verwendung von Verbindungen der allgemeinen Formel I gemäß Anspruch 1 zur Herstellung von GADOBUTROL der Formel II

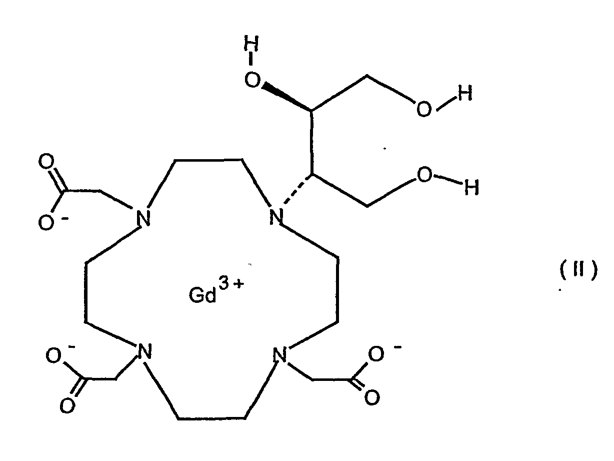

4. Verbindungen der allgemeinen Formel I gemäß Anspruch 1, **dadurch gekennzeichnet, dass** Y für Chlorid, ein X für ein Lithiumion und die beiden restlichen X für Wasserstoffatome stehen.

## Claims

1. Compounds of general formula I 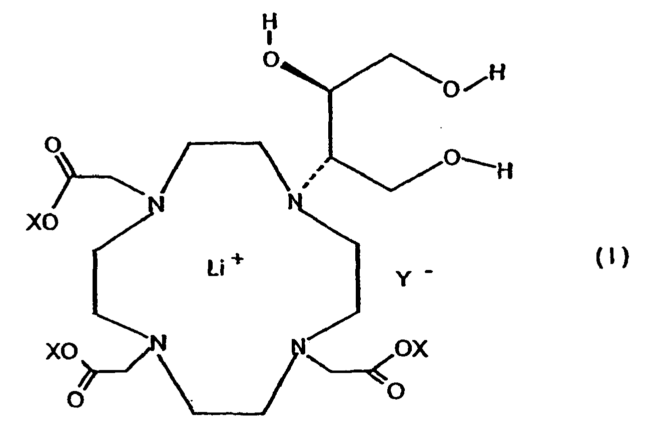 in which the three X altogether stand for n lithium ions and m hydrogen atoms, whereby n and m in each case mean the numbers 0 to 3, preferably 0.8-2.2, and the sum of n and m = 3, and Y has the meaning of chloride and bromide.

2. Process for the production of compounds of general formula I according to claim 1, **characterized in that** N-(6-hydroxy-2,2-dimethyl-1,3-dioxepan-5-yl)-1,4,7,10-tetraazacyclododecane or its complex is reacted with lithium chloride or lithium bromide with chloroacetic acid or bromoacetic acid and lithium hydroxide in polar solvents at temperatures of between 40 and 150°C and a pH of between 8 and 14.

3. Use of the compounds of general formula I according to claim 1 for the production of GADOBUTROL of formula II

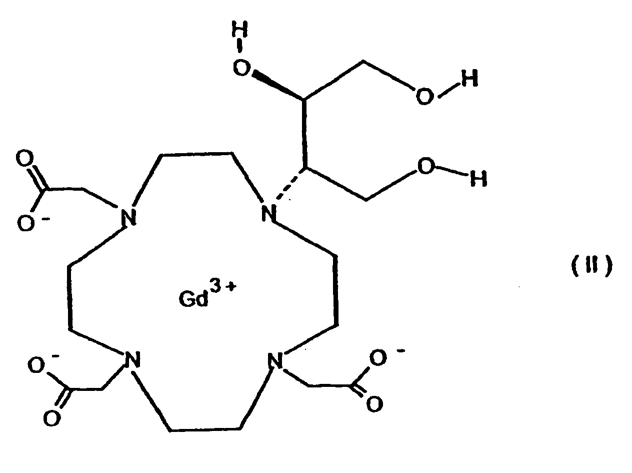

4. Compounds of general formula I according to claim 1, wherein Y stands for chloride, and X stands for a lithium ion, and the two remaining X stand for hydrogen atoms.

## Revendications

1. Composés de formule générale 1 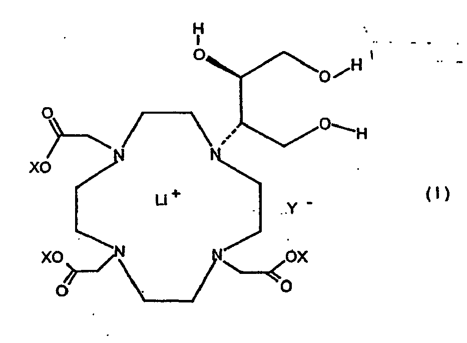 dans laquelle les trois groupes X représentent au total n ions lithium et m atomes d'hydrogène, où n et m représentent chacun les nombres 0 à 3, de préférence 0,8 à 2,2, et la somme de n et m vaut 3, et Y représente un groupe chlorure et un groupe bromure.

2. Procédé de préparation de composés de formule générale I selon la revendication 1, **caractérisé en ce que** du N-(6-hydroxy-2,2-diméthyl-1,3-dioxépan-5-yl)-1,4,7,10-tétraazacyclododécane ou son complexe avec du chlorure de lithium ou du bromure de lithium est mis à réagir avec de l'acide chloroacétique ou bromoacétique et de l'hydroxyde de lithium dans des solvants polaires à des températures comprises entre 40 et 150 °C et un pH compris entre 8 et 14.

3. Utilisation de composés de formule générale I selon la revendication 1 pour la préparation de GADOBUTROL de formule II

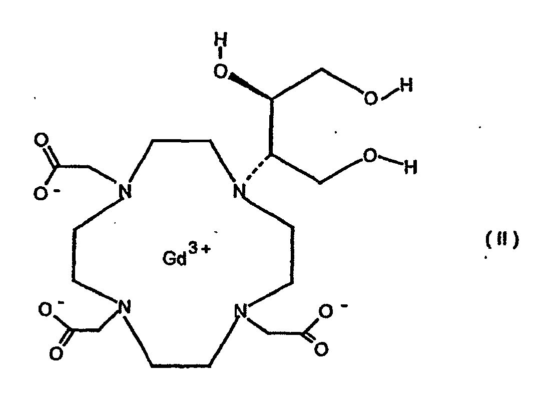

4. Composés de formule générale I selon la revendication 1, **caractérisé en ce que** Y représente un groupe chlorure, un groupe X représente un ion lithium et les deux groupes X restants représentent des atomes d'hydrogène.
